# EUROPEAN PATENT SPECIFICATION

(11) **EP 1 406 507 B1**
(45) Date of publication and mention of the grant of the patent: **03.01.2007**
(21) Application number: 02745586.4
(22) Date of filing: 05.07.2002
(51) Int. Cl.: A23J 1/20, A23L 1/305

(54) **METHODS OF EXTRACTING CASEIN FRACTIONS FROM MILK AND CASEINATES AND PRODUCTION OF NOVEL PRODUCTS**
VERFAHREN ZUR GEWINNUNG VON KASEINFRAKTIONEN AUS MILCH UND KASEINATEN UND HERSTELLUNG NEUER PRODUKTE
PROCEDES PERMETTANT D'EXTRAIRE DES FRACTIONS DE CASEINE DU LAIT ET DE CASEINATES ET PRODUCTION DE NOUVEAUX PRODUITS

(30) Priority: 06.07.2001 GB 0116509
(43) Date of publication of application: 14.04.2004
(73) Proprietor: THE HANNAH RESEARCH INSTITUTE, Ayr, Ayrshire KA6 5HL (GB)
(72) Inventor: LAW, Andrew, Ayr, Ayrshire KA6 5HL (GB); LEAVER, Jeff, Ayr, Ayrshire KA6 5HL (GB)
(74) Representative: McKechnie, Neil Henry
(86) International application number: PCT/GB2002/003098
(87) International publication number: WO 2003/003847

(56) References cited:
- EP-A- 0 393 850
- WO-A-94/06306
- WO-A-94/15952
- WO-A-98/08402
- WO-A-98/14071
- FR-A- 2 592 769
- US-A- 5 216 129
- US-A- 5 397 577
- WAUGH D.E. AND VON HIPPEL P.H.: "kappa-Casein and the Stabilization of Casein Micelles" JOURNAL OF THE AMERICAN CHEMICAL SOCIETY, vol. 78, 1956, pages 4576-4582, XP002215654 cited in the application
- MCKENZIE H.A. AND WAKE R.G.: "An improved method for the isolation of kappa-casein" BIOCHIMICA ET BIOPHYSICA ACTA, vol. 47, 1961, pages 240-242, XP008008899 cited in the application
- CHRISTENSEN T M I E ET AL: "QUANTITATIVE FRACTIONATION OF CASEIN BY PRECIPITATION OR ION EXCHANGE CHROMATOGRAPHY" MILCHWISSENSCHAFT, VV GMBH VOLKSWIRTSCHAFTLICHER VERLAG. MUNCHEN, DE, vol. 44, no. 8, 1989, pages 480-484, XP000036830 ISSN: 0026-3788
- MAUBOLS J-L ET AL: "MILK PROTEIN FRACTIONATION" INTERNATIONAL DAIRY FEDERATION SPECIAL ISSUE, INTERNATIONAL DAIRY FEDERATION.BRUSSELS, BE, vol. 9201, 1992, pages 15-22, XP001015484 ISSN: 1025-8515

## Description

### Introduction

The present invention relates to a novel method for selectively precipitating casein fractions from skim-milk or caseinates by the addition of a calcium salt at alkaline pH.

The invention relates in particular to the preparation of pure fractions of αs-casein and β-casein, and for the first time a fraction highly enriched in κ-casein. The products are suitable for use in food or for preparing novel food materials and the method can be used on an industrial scale. The purified casein fractions have distinctive properties and have a wide range of uses, including the stabilisation of emulsions and foams, the preparation of infant formula or preparations suitable for use by sufferers of metabolic disorders, such as phenylketonuria (PKU), and the manufacture of edible coatings and biodegradable plastics. The fractions are also particularly suitable as starting materials for processes involving limited proteolysis to produce new cheese flavours, nutraceuticals and a wide range of bioactive peptides. These include phosphopeptides and other bioactive peptides with antimicrobial, antihypertensive, immunomodulatory, antithrombotic and opiod functions.

Dairy products form a major part of the human diet, the total World production of cows' milk in 1998 being about 57 million tonnes. Milk itself is a relatively complex mixture, the main constituents being fat, minerals, sugar (lactose) and protein. The proteins are present in milk at a concentration of about 35 g/L, and can be conveniently divided into two major classes - the caseins, which are retained in the curd, and the whey proteins, which remain in the residual liquid during cheese manufacture.

The caseins account for 80% of the protein in milk and occur, together with much of the calcium and phosphate, in sub-micron-sized colloidal particles, which are termed the casein micelles. Scattering of light by the casein micelles gives milk its characteristic white appearance. The integrity of the casein micelles is crucial to the stability of milk and the properties of products made from milk are in part determined by the properties of the micelles. For example, it is known that the firmness of cheeses can be directly correlated with the average diameter of the micelles in the milk from which they are made. Worldwide cheese production was estimated to be in the region of 15 million tonnes in 1998. Increasingly, milk proteins are consumed not as liquid milk but as ingredients in products that have been processed to a greater or lesser extent. The degree of processing ranges from yoghurt and cottage cheese manufacture, in which the micelles are induced to aggregate by acidification, to the incorporation of milk protein fractions into sauces and spreads.

When milk is acidified to pH 4.6, calcium phosphate dissolves from the casein micelles and the micellar structure is disrupted, causing the casein to aggregate and precipitate. This procedure is used in the manufacture of sodium caseinate, which is prepared by re-suspending the acid casein precipitate in aqueous solution by the addition of sodium hydroxide to about pH 7.0, followed by drying. Other forms of caseinate with different functional properties, such as calcium caseinate or ammonium caseinate, can be obtained by adjusting the casein to pH 7.0 with the appropriate calcium or ammonium hydroxide. Annual world-wide production of casein and caseinates is in the region of 250,000 tonnes. Caseinates are used in the manufacture of a wide variety of processed foods where use is made of their excellent foaming, emulsifying and water-binding properties.

The casein in cows' milk is made up of four different proteins, namely κ-, β-, αₛ₁- and αₛ₂-caseins. The composition of whole casein, on average, is κ-, 11.0 %; β-, 35.0 %; αₛ₁-, 36.0 % and αₛ₂-, 10.0 %, the remainder being breakdown products of the major caseins. The individual caseins have fairly similar molecular weights and isoelectric points, and have high affinities for Ca²⁺ and inorganic phosphate, but they have completely different amino acid sequences and properties.

After biosynthesis, the caseins all undergo phosphorylation at serine and occasionally threonine amino acid residues in the characteristic tri-peptide sequences -Ser/Thr-X-A, where X represents any amino acid residue and A is an acidic amino acid residue such as Asp, Glu, SerP or ThrP. These phosphate centres are important in that they are involved in binding calcium phosphate and in maintaining the casein micellar structure. In this way, both protein and calcium phosphate, which are important in nutrition, are maintained at stable high concentrations in milk. The αₛ₁- and αₛ₂-caseins have the most serine phosphate groups in their structure, the highest net negative charges at alkaline pH, and the greatest affinities for Ca²⁺. β-Casein is intermediate in these respects, whereas κ-casein has only one serine phosphate group, the lowest negative charge and the least affinity for Ca²⁺.

The caseins as a group are very hydrophobic and, when the micellar structure is disrupted, have a high tendency to aggregate in aqueous solution, β-casein being most hydrophobic, the αₛ₁- and αₛ₂-caseins intermediate, and κ-casein the least hydrophobic. κ-Casein also differs from the other caseins in that it occurs in milk as a series of disulphide-linked polymers, and contains carbohydrate side-chains.

The caseins have an open structure and readily undergo proteolysis but they show different susceptibilities to a wide range of proteolytic enzymes. For example, β- and αₛ₂-caseins are more readily attacked than the other caseins by plasmin which is present in milk. κ-Casein also readily undergoes specific proteolysis with chymosin, and this forms the basis of the renneting process that occurs during cheese-manufacture. The hydrophilic part of κ-casein that protrudes from the surface of the micelles and normally maintains their stability, is split off, allowing the remainder of the casein to aggregate and form the curd. The fragment of κ-casein split off into the whey - the caseinomacropeptide (CMP) - has distinctive properties and, as it does not contain aromatic amino acids, is useful as a dietary protein for sufferers of the genetic metabolic disorder phenylketonuria (PKU).

Specifically, the κ-casein fraction is unusual because it is broken down in a specific way by the enzyme chymosin into two well defined parts - para κ-casein and CMP. CMP has been reported to have useful pharmacological properties. In particular, it contains no aromatic amino acids, and specifically it contains no phenylalanime. This is particularly significant because CMP is a potential source of essential protein for patients suffering from PKU. PKU is a metabolic condition where subjects are unable to metabolise the aromatic amino acids phenylalanime. The condition is such that if phenylalanime is consumed, it accumulates in the blood stream and brain tissue and can ultimately cause severe mental disorders. The condition is serious and all infants in the UK are routinely screen soon after birth to identify the condition, and to take steps to modify the diet to exclude phenylalanime. However, few natural proteins are known to be completely free from this amino acid. As a result, the dietary requirements of those suffering from PKU are met by a mixture of amino acids from which phenylalanime has been removed by specific enzymic treatment. These mixtures are relatively effective as nutrients, but are expensive to manufacture and unpalatable.

However, CMP, as manufactured from the κ-casein fraction described herein offers a practical alternative source of essential amino acids without the concomitant danger of ingestion of phenylalanime.

### Prior art techniques for fractionating caseins

In milk, the four individual caseins, κ-, β-, αₛ₁- and αₛ₂-caseins, are closely associated with microgranules of calcium phosphate in micellar form. In order to fractionate the caseins, it is necessary to at least partially disrupt the micellar structure by some combination of acidification, renneting, cooling or addition of a chelating agent. The caseins are hydrophobic in nature and, once dissociated from the micelles, have to be prevented from re-aggregation by the addition of a chaotropic agent or cooling. The individual caseins can then be separated on the basis of the differences in their net negative charge, affinity for Ca, or hydrophobicity.

The technique most commonly used on a laboratory scale for separating the four major caseins is anion-exchange chromatography. Resolution of the individual caseins depends mainly on differences in the net negative charges of the proteins at alkaline pH, and on their affinity for a positively charged column medium. All of the separations require that casein is dissociated in high concentrations of a chaotropic medium such as urea (>3.3 M), and treated in the presence of a reducing agent such as 2-mercaptoethanol to reduce the disulphide linkages in the series of κ-casein polymers (JOURNAL OF DAIRY SCIENCE, vol. 49, 1966, pages 792-795. Thompson, M.P. "DEAE-Cellulose-urea chromatography of casein in the presence of 2-mercaptoethanol"). Resolution and flow characteristics of the column media have improved over the years but the technique is not easily scaled up. Also, even on exhaustive dialysis of the fractions, some residual toxic materials are present, and the casein fractions are not suitable for use in foods.

Separation of the major caseins from whole casein has also been achieved on a laboratory scale using cation-exchange chromatography in which separation is based on the net positive charge of the caseins at acid pH and their binding to a negatively charged column medium (JOURNAL OF DAIRY RESEARCH vol. 36, 1969, pages 259-268. Annan, W.D. and Manson, W. "A fractionation of the αₛ-casein complex of cows' milk"; JOURNAL OF DAIRY RESEARCH vol. 59, 1992, pages 557-561. Leaver, J. and Law, AJR. "Preparative-scale purification of bovine caseins on a cation-exchange resin"). Cation-exchange chromatography is not easily scaled up, and requires the presence of high concentrations of a dissociating agent such as urea (>3.3 M), and a prior reduction treatment with a material such as 2-mercaptoethanol. The casein fractions are not, therefore, suitable for human consumption.

Another laboratory method occasionally used for partial fractionation of the caseins involves the addition of high concentration of urea, as a chaotropic agent, to whole casein to bring about dissociation of the individual caseins. A crude separation of αₛ-, β- and κ- casein components is then obtained on the basis of differential solubility in urea (JOURNAL OF DAIRY CHEMISTRY vol. 46, 1963, pages 1183-1188. Zittle, CA. And Custer, J.H. "Purification and some properties of αₛ-casein and κ-casein). Due to the very high concentration of urea (6.6 M) required to dissociate the casein, it is impractical to perform these separations economically on a large scale, and the products are not suitable as food materials.

A crude fractionation of the major caseins can also be obtained according to their solubilities in ethanol in the presence of various salts (JOURNAL OF DAIRY SCIENCE vol. 35, 1952, pages 272-281. Hipp, N.J., Groves, M.L., Custer, J.H. and McMeekin, T.L. "Separation of α-, β- and γ-casein"). Refinements of this procedure have been used to obtain purified fractions of κ-casein from mixtures containing αₛ-and β-caseins (BIOCHIMICA BIOPHYSICA ACTA vol. 47, 1961, pages 240-242. McKenzie, H.A. and Wake, R.G. "An improved method for the isolation of κ-casein"; BIOCHEMISTRY vol. 9, 1970, pages 2807-2813. Talbot, B and Waugh, D.F. "Micelle-forming characteristics of monomeric and covalent polymeric κ-caseins"). These methods require high concentrations of ethanol, and most of the salts used are toxic. β-Casein is more hydrophobic than the other major caseins and tends to dissociate at low temperatures when hydrophobic interactions are weaker, even at its isoelectric point. β-Casein, therefore, can be prepared from a solution of sodium caseinate by isoelectric precipitation of the other caseins at pH 4.5 and 2°C, and precipitation of the β-casein from the supernatant on warming to 20°C (JOURNAL OF AMERICAN CHEMICAL SOCIETY vol. 67, 1944, pages 1725-1731. Warner, RC. "Separation of α- and β-casein").

Cooling milk, particularly at acid pH causes dissociation of some of the caseins, particularly β-casein, from the micelles. The β-casein enriched liquid phase can then be separated from the other β-casein-depleted phase. This cold-induced solubilisation of β-casein, starting from rennet casein (the solid obtained in the initial step of cheese making), has been reported (Patent number WO9406306, "A process for producing beta-casein enriched products". Ram, S., Loh, DW., Love, DC. and Dudley, EP.; Patent number US5397577 "Method for obtaining beta casein" Le Magnen, C. and Maugas, J-J.).

Patent US5397577 describes a process for extracting β-casein from a rennet casein suspension or solution by acidifying to pH 4 to 5 and cooling to -2 to 10 °C. Two phases form of which the liquid phase contains β-casein and the solid phase is a mixture of the other αₛ₁- and κ-caseins and is substantially free of β-casein.

Patent WO9406306 describes a process for the partial extraction of β-casein by holding a slurry of casein feedstock in the cold at a pH between 3.5 and 8.0 for a suitable time and separating said slurry into two phases to obtain a solid phase which is β-casein depleted and a liquid phase which is β-casein enriched.

Patent FR2592769 ("Process for producing a material enriched in beta-casein, apparatus for implementing this process, and application of the products obtained by this process as foodstuffs, food supplements or additives in the food and pharmaceutical industries." Terre, E., Maubois, J-L., Brule, G. and Alice, P.) describes a process for the production of a β-casein enriched fraction and a correspondingly depleted fraction by either treating milk with a calcium complexing agent or treating a caseinate solution with an agent which polymerises all of the casein and cooling to 0 to -7 °C and subjecting the material to microfiltration on an inorganic membrane under tangential flow. The microfiltrate material is enriched in β-casein and the retentate is depleted. Microfiltration at low temperature is very slow, making the process less attractive industrially.

A partial separation of the caseins can be obtained on a laboratory scale by the addition of calcium chloride at neutral pH to milk or caseinate (JOURNAL OF AMERICAN CHEMICAL SOCIETY vol. 78, 1956, pages 4576-4582. Waugh, DF and von Hippel, PH. "κ-Casein and the stabilization of casein micelles"; BIOCHIMICA BIOPHYSICA ACTA vol. 47, 1961, pages 240-242. McKenzie, HA and Wake, RG. "An improved method for the isolation of κ-casein"). Both methods give a precipitate of αₛ- and β-caseins and a supernatant containing a mixture of κ-, αₛ- and β-caseins. These methods suffer from the disadvantages that they require high speed centrifugation to separate the fine precipitate from the supernatant, and dialysis to remove excess calcium chloride from the κ-casein enriched fraction, and cannot therefore be easily scaled up. The use of high concentrations of calcium chloride makes recovery of the κ-casein enriched fraction difficult, as the casein will not undergo the normal isoelectric precipitation. Also, the addition of potassium oxalate, which is toxic, to remove excess calcium precludes the use of this fraction as a food material.

Thus, previous methods of casein fractionation involving selective calcium precipitation have been carried out at neutral pH (JOURNAL OF AMERICAN CHEMICAL SOCIETY vol. 78, 1956, pages 4576-4582. Waugh, DF and von Hippel, PH. "κ-Casein and the stabilization of casein micelles"; BIOCHIM. BIOPHYS. ACTA vol. 47, 1961, pages 240-242. McKenzie, HA and Wake, RG. "An improved method for the isolation of κ-casein"). These methods describe partial separation of the caseins obtained by the addition of calcium chloride to milk or caseinate, respectively, near 7.0. Both methods required higher concentrations of calcium chloride (0.25-0.36M) than in the present alkaline precipitation (0.059-0.071M), and precipitation of the αₛ- and β-caseins did not occur readily, but instead required high speed centrifugation. Also, the high concentration of calcium chloride in the supernatant prevented subsequent acid precipitation of the κ-casein fraction, and the addition of oxalate to chelate calcium and dialysis were required before the protein could be recovered. It was, therefore, not possible to scale up the methods, and the products were not suitable for human consumption.

Due to the hydrophobicity of the individual casein components, it is difficult to fractionate the caseins on a large scale, even though each of the proteins has specific functional properties that may be interesting to the food and pharmaceutical industries (PROCEEDINGS OF THE 25^{th} INTERNATIONAL DAIRY CONGRESS, 1998, pages 74-86. Maubois, J.-L. "Fractionation of milk proteins"). At present, therefore, caseins tend to be used as a mixture of the four proteins rather than as individual components. A simple, inexpensive method of fractionating this mixture of proteins would be beneficial since it would enable food and pharmaceutical manufacturers to use ingredients that are currently so expensive as to preclude their use on anything greater than a laboratory scale. The aim of the present invention is to provide a method that can be used on an industrial scale for the production of purified individual casein fractions in a form suitable for use in food or novel food products or in the preparation of nutraceuticals, phosphopeptides and other bioactive peptides.

The present invention involves fractionation of the caseins by addition of a calcium chloride to milk or caseinate at alkaline pH. The advantages of carrying out the selective precipitation at alkaline pH are that the calcium sensitive αₛ- and β-caseins sediment more readily, and can be obtained without high-speed centrifugation. Also, a lower concentration of calcium salt is required at alkaline pH, and the κ-casein enriched fraction can be obtained by acid precipitation without the use of toxic chelating agents or dialysis. The present procedure, unlike that at neutral pH, can be carried out on an industrial scale, and the products are suitable for use in food and novel food materials and as starting materials for preparing nutraceuticals, phosphopeptides, and a wide range of bioactive peptides.

In addition, the process described in this application allows effective practical and inexpensive separation of CMP from mixtures of whey protein, casein and proteose peptones found in milk and whey. Conventional methods of manufacturing CMP use κ-casein preparations which contain significant impurities. For example, if a κ-casein preparation is manufactured from skimmed milk, the preparation contains cold denatured β-lactoglobulin. On the other hand, if a κ-casein preparation is made using whole casein (typically of sodium or potassium salt), the preparation contains a small proportion of αₛ-and β-casein. During hydrolysis of the κ-casein by chymosin to form CMP limited proteolysis contaminants might occur. As a result, there is a danger that the final preparation may be contaminated by peptides containing phenylalanime, thus rendering the preparation unsuitable for ingestion by those suffering from PKU.

The aims of the present invention therefore include:
- Devising a new method of extracting purified casein fractions and β-lactoglobulin from milk, yielding not only the protein fractions but an acid whey partially depleted in β-lactoglobulin. The method can be used on an industrial scale, and the protein fractions and the acid whey are suitable for use in food or for preparing novel food materials such as humanised infant formula, cheese flavours, emulsions, edible coatings and nutraceuticals. The purified fractions can also be used to prepare biodegradable plastics, phosphopeptides and other bioactive peptides.
- Devising a new method of extracting purified casein fractions from caseinate. The method can be used on an industrial scale, and the casein fractions are suitable for use in food, novel food products, nutraceuticals and biomedical applications as mentioned above.
- Devising a new method of manufacturing CMP, suitable for use by sufferers of the metabolic disorder phenylketonuria.

Within this document, references to casein micelles, caseins, caseinates and other components of milk should be interpreted, unless context requires otherwise, as including all the related inter- and intra-species variants of these components. β-Casein, for example, will have a slightly different primary structure in different mammals; it will be clear to one skilled in the art that the invention will apply to all these variants. The term aₛ-casein refers to a mixture of aₛ₁- and aₛ₂-caseins.

According to a first aspect of the present invention there is provided a method of obtaining purified protein fractions from milk including the steps of:
i) increasing the pH of the milk to about pH 11;
ii) adding Calcium Chloride to selectively precipitate the fractions;
iii) decreasing the pH; and
iv) carrying out centrifugation or filtration to separate the fractions.

Preferably the fractions are αₛ-, β- and κ- casein.

Typically the milk is skim milk.

Preferably centrifugation is carried out at low speed.

The method may further comprise the step of carrying out precipitation at acidic pH of the supernatant obtained from the centrifugation or filtration carried out in step (iv), to obtain a fraction highly enriched in κ-casein.

In the step where the κ-casein enriched fraction is obtained by adjusting the supernatant to acid pH, preferably the pH is 3.8. Typically the content of κ-casein is about 60%, and that of β-lactoglobulin about 20 %.

The method may also comprise the steps of re-dissolving the αₛ- and β-casein components in water, and carrying out repeated selective isolelectric precipitation of the αₛ-casein at acidic pH and low temperature, to obtain a fraction containing mainly αₛ-casein.

In the step where the αₛ- and β-casein precipitate are re-dissolved in water, preferably the water is at pH 7.0 and 20°C.

The solution of αₛ- and β-caseins may be cooled after being re-dissolved in water, preferably to 2°C, and adjusted to acidic pH, preferably to pH 4.5.

The procedure of re-dissolving and re-precipitation may be repeated, preferably a further two times, yielding a fraction containing mainly αₛ-casein (more than 85%) and a fraction containing mainly β-casein (more than 95%).

Yet further the method may also comprise the steps of warming the supernatant obtained from the selective isoelectric precipitation, and carrying out acid precipitation to recover the fraction containing mainly β-casein. The supernatant acid whey is partially depleted in β-lactoglobulin, but contains all the other whey proteins in undenatured form, and is enriched in calcium.

Preferably the supernatant is warmed to 35°C.

Preferably the pH of the milk is increased by the addition of an alkaline solution and decreased by the addition of an acid solution.

Preferably, in the step where the pH of the milk is increased, sodium hydroxide is added.

Preferably the temperature of the milk on addition of the alkaline solution is 30°C.

Optionally, after adding the alkaline solution, the milk may be allowed to stand for a few minutes. Preferably the milk is left to stand for less than 5 min.

Preferably the final concentration of calcium chloride in the alkaline caseinate solution is 0.059 M.

The pH may be decreased by addition of one or more mineral acids.

The method may also comprise the step of increasing the pH of fraction highly enriched in κ-casein and carrying out ultra-filtration to separate the low molecular weight material from the caseins, whey proteins and caseinomacropeptide.

The pH will typically be raised to a neutral value.

Preferably ultra-filtration is carried out using a membrane with a pore size less than 25kD, but greater than 12kD.

Yet further, the method may also comprise the step of lowering the pH of the fraction highly enriched in κ-casein and carrying out ultra-filtration to separate the whey protein from the caseinomacropeptide.

The pH is typically lowered to between pH4 and pH5.

Preferably the pH is lowered to pH4.6.

Preferably ultra-filtration is carried out using a membrane with a pore size more than 7kD, but less than 20kD.

According to a second aspect of the present invention there is provided a method of obtaining purified protein fractions from a solution of sodium caseinate including the steps of:
ii) increasing the pH of the sodium caseinate solution to about pH 11;
iii) adding Calcium Chloride to selectively precipitate the fractions;
iv) decreasing the pH; and
v) carrying out centrifugation or filtration to separate the fractions.

Preferably centrifugation is carried out at low speed.

The method may also comprise the step of carrying out precipitation at acidic pH of the supernatant obtained from the centrifugation or filtration in step (iv) to obtain a fraction highly enriched in κ-casein.

In the step where the κ-casein enriched fraction is obtained by adjusting the supernatant to acid pH, preferably the pH is 3.8. The κ-casein content of this fraction would usually be greater than 30%.

Optionally the fraction enriched in κ-casein may be further enriched in κ-casein by selective precipitation from ethanol The κ-casein fraction may be re-dissolved in water at pH 7.0, re-precipitated at pH 4.6, and then selectively precipitated from 50% ethanol at pH 7.0 and 25°C with the addition of a small amount of 2M NaCl in 50% ethanol. The κ-casein precipitate forms readily and may be removed by filtration or low speed centrifugation. (BIOCHEMISTRY vol. 9, 1970, pages 2807-2813. Talbot, B. and Waugh, D.F. "Micelle-forming characteristics of monomeric and covalent polymeric κ-caseins"). The κ-casein content of this fraction would typically be about 65%.

The method may further comprise the steps of re-dissolving the αₛ- and β-casein components in water and carrying out repeated selective isoelectric precipitation of the αₛ-casein at acidic pH and low temperature to obtain a fraction containing mainly αₛ-casein.

In the step where the αₛ- and β-casein precipitate is re-dissolved in water, preferably the water is at pH 7.0 and 20°C.

The solution of αₛ- and β-casein may be cooled, preferably to 2°C, and adjusted to acid pH, preferably to pH 4.5.

Preferably the method also comprises the steps of warming the supernatant obtained from the selective isolelectric precipitation, and carrying out acid precipitation to recover the fraction containing mainly β casein. β-Casein may be precipitated from the supernatant by warming, preferably to 35°C.

The procedure of re-dissolving and re-precipitation is repeated, preferably a further two times, yielding a fraction containing mainly αₛ-casein (more than 85%) and a fraction containing mainly β-casein (more than 95%).

Preferably the pH of the sodium caseinate solution will be increased by the addition of an alkaline solution and decreased by the addition of an acid solution.

More preferably, in the step where the pH of the sodium caseinate solution is increased, the pH may be raised to 11 by addition of sodium hydroxide.

Preferably the temperature of the sodium caseinate solution on addition of the alkaline solution is 30°C.

The increased pH sodium caseinate solution may be allowed to stand for a few minutes, preferably less than 5 min.

Preferably the final concentration of calcium chloride in the alkaline sodium caseinate solution will be 0.071 M.

The pH may be decreased to 7.0 by addition of one or more mineral acids.

The method may also comprise the step of increasing the pH of the fraction highly enriched in κ-casein and carrying out ultra-filtration to separate the low molecular weight material from the caseins, whey proteins and caseinomacropeptide.

The pH will typically be raised to a neutral value.

Preferably ultra-filtration is carried out using a membrane with a pore size less than 25kD, but greater than 12kD.

Yet further, the method may also comprise the step of lowering the pH of the fraction highly enriched in κ-casein and carrying out ultra-filtration to separate the whey protein from the caseinomacropeptide.

The pH is typically lowered to between pH4 and pH5. Preferably the pH is lowered to pH4.6.

Preferably ultra-filtration is carried out using a membrane with a pore size more than 7kD, but less than 20kD.

The caseinomacropeptide may be concentrated and dried. Typically this is carried out by freeze spray drying.

The invention will now be described with reference to the following figures in which:

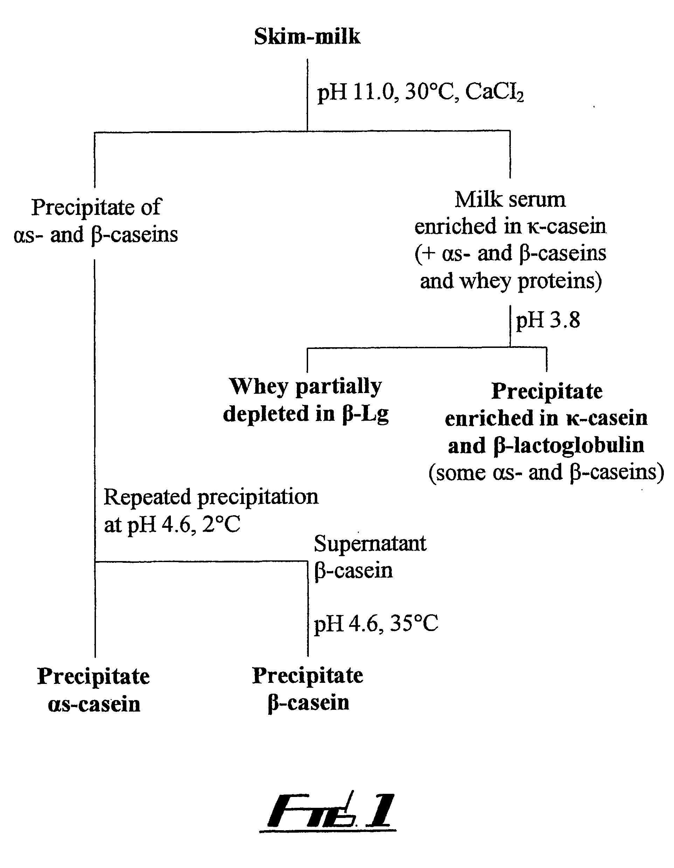

Figure 1 is a diagram illustrating the steps involved in fractionating the casein in skim-milk into αₛ-casein, β-casein, and a fraction enriched in κ-casein and β-lactoglobulin (β-Lg), containing some αₛ- and β-caseins. The procedure also yields an acid whey partially depleted in β-lactoglobulin, as detailed in example 1;

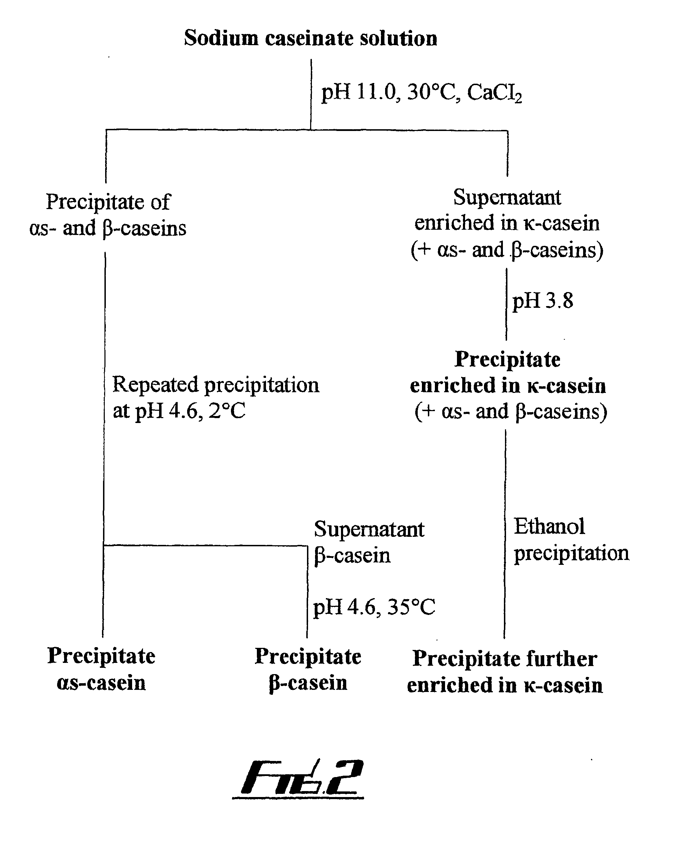

Figure 2 is a diagram illustrating the steps involved in fractionating sodium caseinate into αₛ-casein, β-casein and a fraction enriched in κ-casein, containing αs- and β-caseins as detailed in example 2;

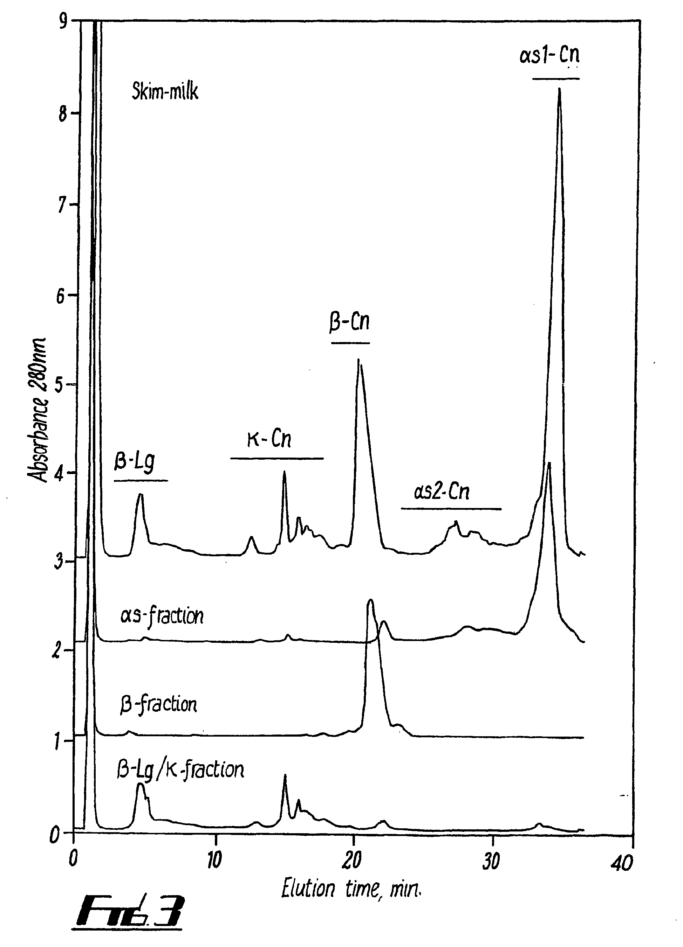

Figure 3 shows the anion-exchange fast protein liquid chromatographic profiles of the original casein in skim-milk and the fractions obtained from it as detailed in example 1. The analysis was carried out as outlined in JOURNAL OF DAIRY RESEARCH vol. 54, 1987, pages 369-376. Davies, D.T. and Law, A.J.R. "Quantitative fractionation of casein mixtures by fast protein liquid chromatography";

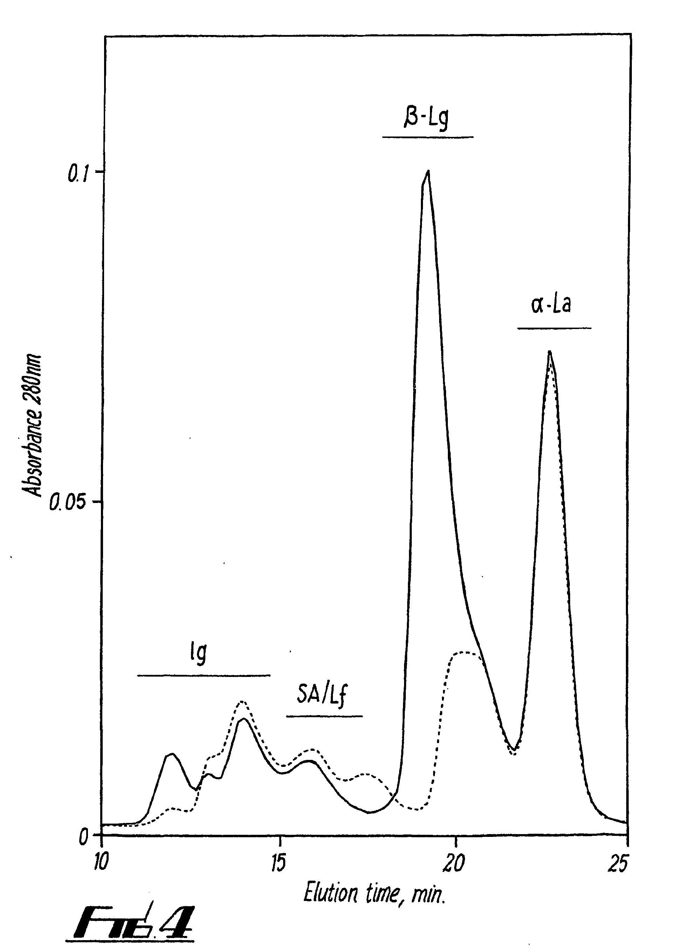

Figure 4 shows the gel permeation profiles of the whey proteins in the original skim-milk (__), and in the supernatant obtained after addition of calcium chloride and after acid precipitation of the κ-casein enriched fraction (---). The figure shows the depletion of β-lactoglobulin due to alkaline denaturation and subsequent precipitation at acid pH. The analysis was carried out as outlined in MILCHWISSENSCHAFT vol. 48, 1993, pages 663-666. Law, A.J.R., Leaver, J., Banks, J.M. and Horne, D.S. "Quantitative fractionation of whey proteins by gel permeation FPLC". (Ig, immunoglobulins; SA/Lf, serum albumin and lactoferrin, β-Lg, β-lactoglobulin; α-La, α-lactalbumin);

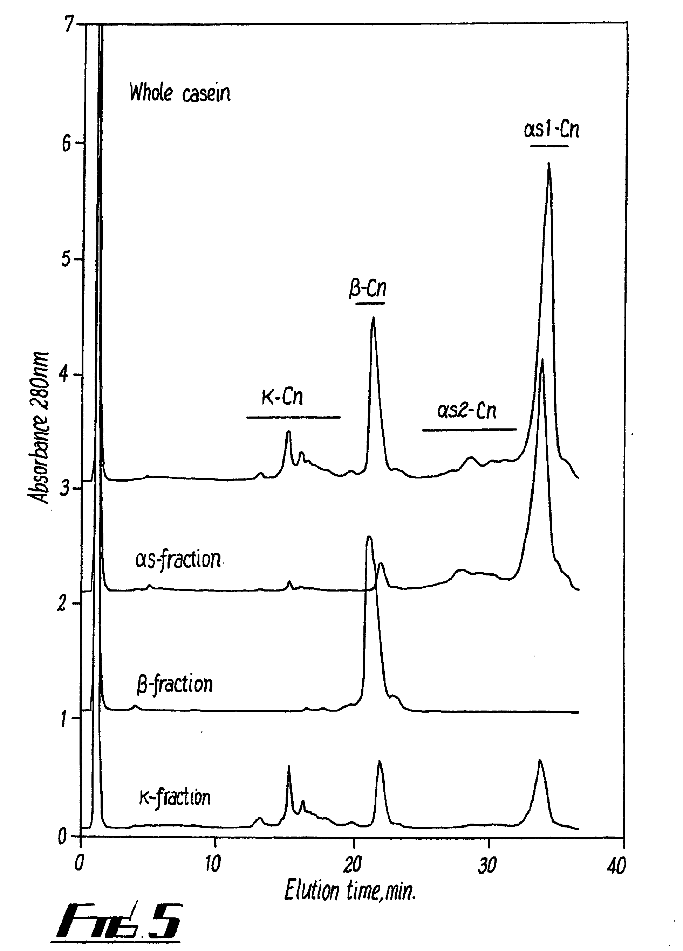

Figure 5 shows the anion-exchange fast protein liquid chromatographic profiles of the original sodium caseinate and the fractions obtained from it as detailed in example 2. Analysis carried out as indicated for Figure 3;

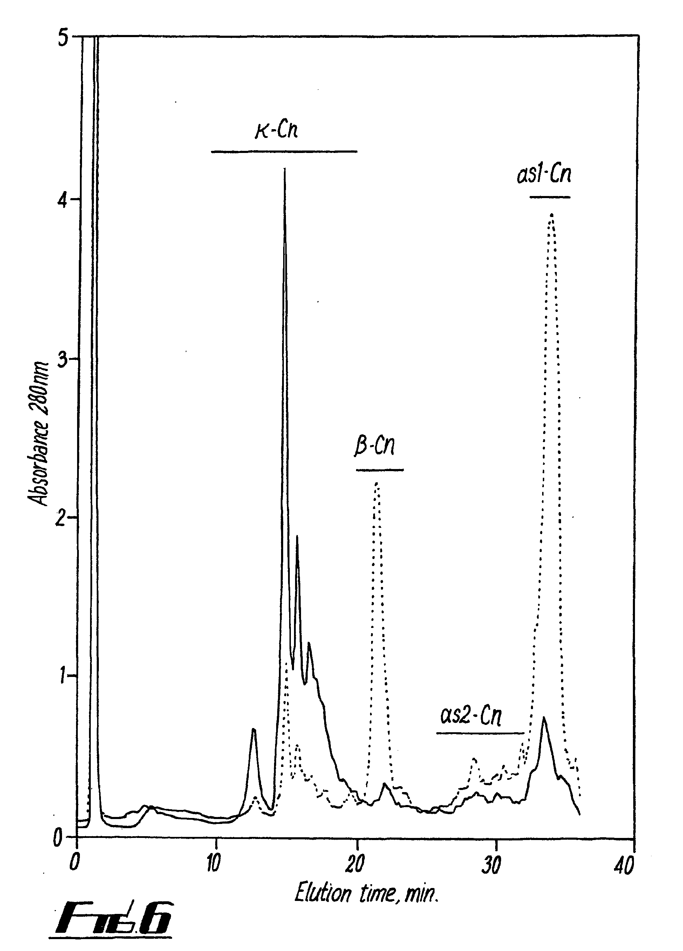

Figure 6 shows the anion-exchange fast protein liquid chromatographic profiles of the original sodium caseinate (---) and the fraction of κ-casein obtained from it (__) as detailed in example 2, after further purification by ethanol precipitation. Analysis carried out as indicated for Figure 3;

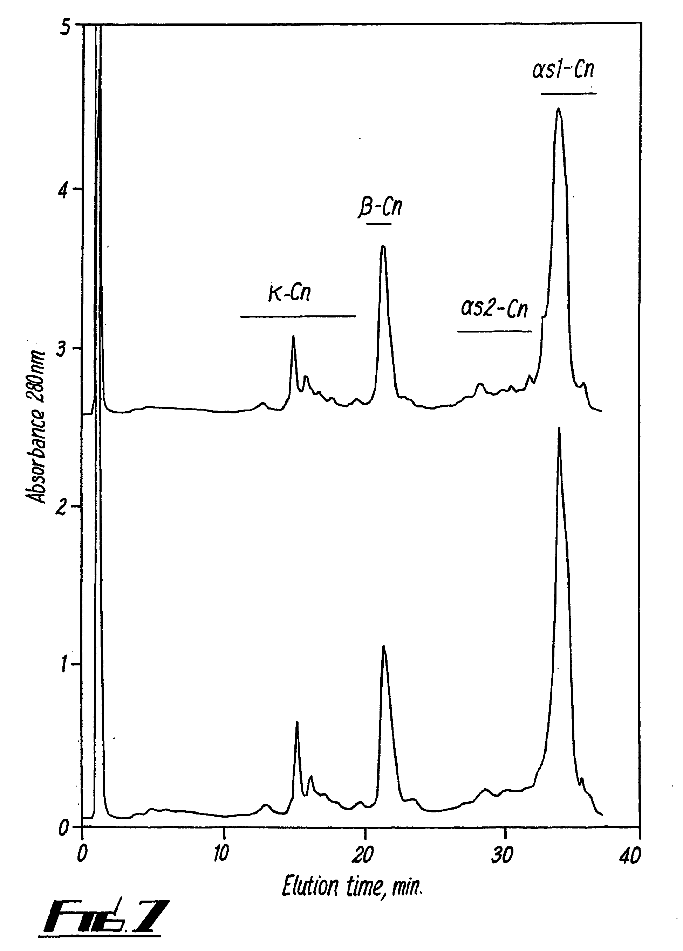

Figure 7 shows the anion-exchange fast protein liquid chromatographic profiles of the original sodium caseinate (top), and sodium caseinate after being held at 30 °C and pH 11.0 for 5.0 min. Analysis carried out as indicated for Figure 3;

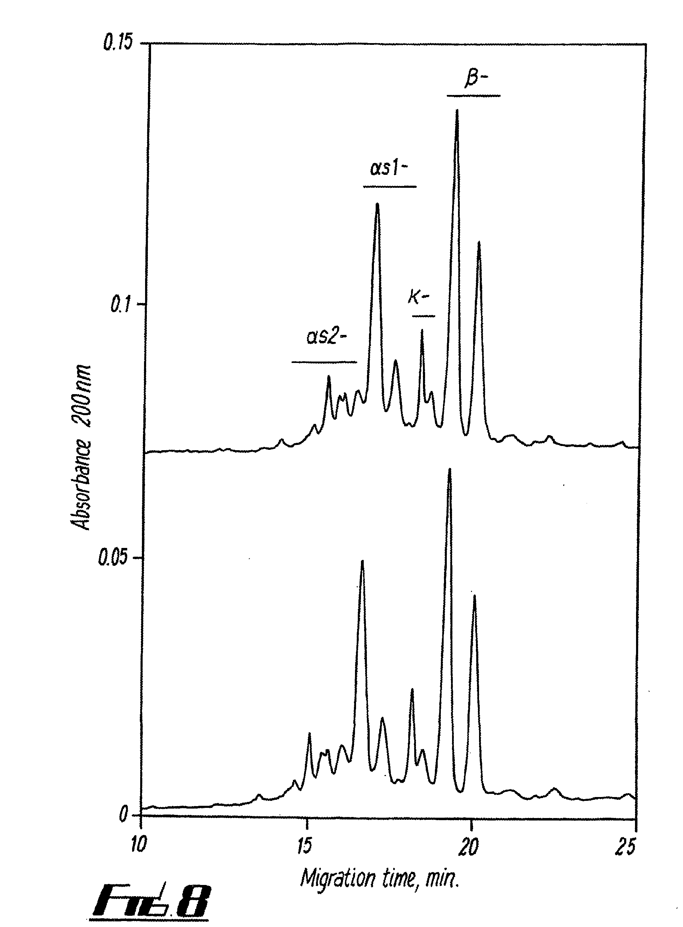

Figure 8 shows the capillary electrophoresis pattern obtained for the original sodium caseinate (top), and sodium caseinate after being held at 30 °C and pH 11.0 for 5.0 min. Analysis essentially according to the method in JOURNAL OF CHROMATOGRAPHY vol. 652, 1993, pages 207-213. de Jong, N., Visser, S. and Olieman, C. "Determination of milk proteins by capillary electrophoresis";

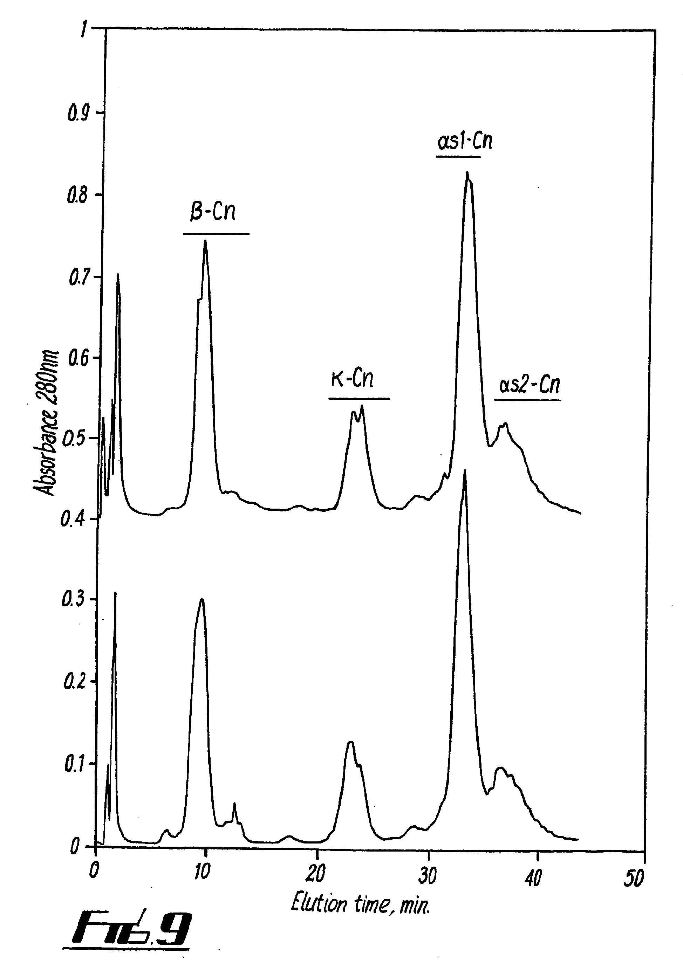

Figure 9 shows the cation-exchange fast protein liquid chromatographic profiles of the original sodium caseinate (top), and sodium caseinate after being held at 30 °C and pH 11.0 for 15.0 min. Analysis was carried out according to the method in JOURNAL OF DAIRY SCIENCE vol. 74, 1991, pages 2403-2409. Hollar, C.M., Law, A.J.R., Dalgleish, D.G. and Brown, R.J. "Separation of major casein fractions using cation-exchange fast protein liquid chromatography"; and

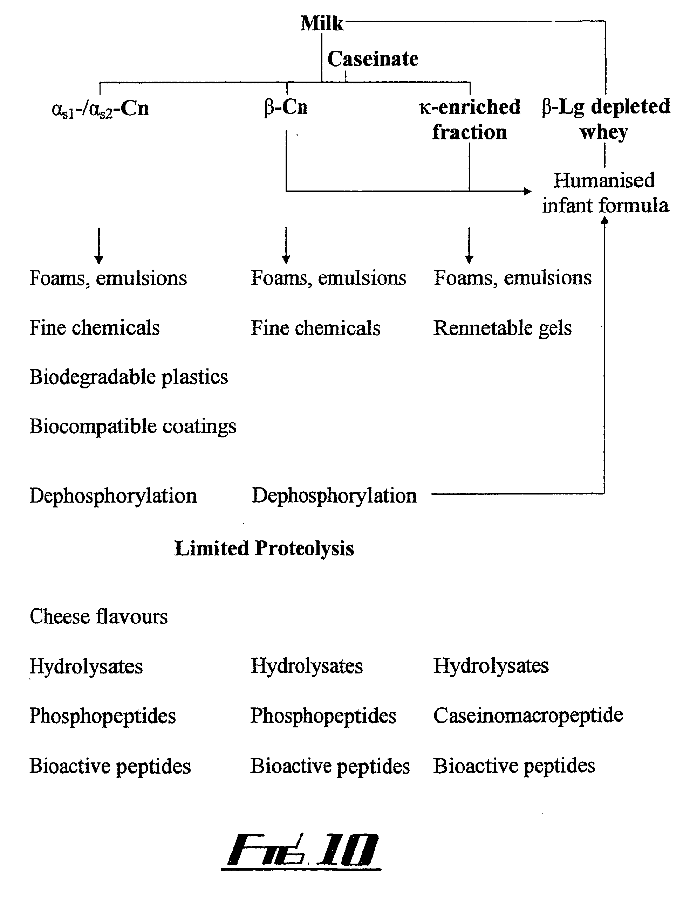

Figure 10 is a diagram illustrating the uses of the purified fractions obtained by selective calcium precipitation from skim-milk or caseinate as outlined in Examples 1 and 2 (Figures 1 and 2, respectively).

The present invention relates to a novel method for selectively precipitating casein fractions from skim-milk or caseinates by the addition of a calcium salt at alkaline pH. The invention relates in particular to the preparation of pure fractions of αs-casein and β-casein, and a fraction highly enriched in κ-casein. The method can be used on an industrial scale, and the products are suitable for use in food or for preparing novel food materials, biodegradable plastics, nutraceuticals and bioactive peptides (Figure 10).

Previous methods of casein fractionation involving selective calcium precipitation have been carried out at neutral pH and require high speed centrifugation, as precipitation of αₛ and β caseins did not occur readily. The higher concentrations of calcium chloride prevented subsequent acid precipitation of the κ-casein fraction, and required the addition of oxalate to chelate calcium, and dialysis before the protein could be recovered. It was, therefore, not possible to scale up the methods, and the products were not suitable for human consumption.

In the present method, casein fractionation is carried out by selective calcium precipitation at alkaline pH. When the pH of skim-milk is increased to about 11.0, the charge on the negatively charged caseins, particularly on the phosphate groups, increases and the caseins dissociate from the micelles. The αₛ-caseins are more highly charged than β-casein, and κ-casein is least highly charged. When a salt such as calcium chloride is added, the αs1- and β-caseins bind more Ca²⁺ than κ-casein does, and readily precipitate leaving the supernatant enriched in κ-casein. At alkaline pH some of the β-lactoglobulin in milk undergoes denaturation (JOURNAL OF THE AMERICAN CHEMICAL SOCIETY vol. 81, 1959, pages 4032-4036. Tanford, C., Bunville, L.G., Nozaki, Y. "The reversible trans-formation of β-lactoglobulin at pH 7.5). The denatured β-lactoglobulin remains in the κ-casein enriched supernatant. When the pH is subsequently reduced to about 3.8, a precipitate enriched in κ-casein readily forms, together with the denatured β-lactoglobulin and some αₛ- and β-caseins (Fig. 3). The acid supernatant is partially depleted in β-lactoglobulin (Figure 4) but contains all the other whey proteins and additional calcium chloride, and is suitable for food use.

Carrying out the selective calcium precipitation at alkaline pH instead of neutral pH has several advantages. At alkaline pH the casein micelles are disrupted and the caseins dissociate without requiring toxic chaotropic or chelating agents. The caseins also have higher net negative charges and, when calcium chloride is added, they bind more Ca²⁺. The caseins can, therefore, be selectively precipitated at lower concentrations of calcium chloride than are required at neutral pH. At alkaline pH, the precipitate of αₛ- and β-caseins readily settles when calcium chloride is added, and can be easily separated by filtration or low speed centrifugation. The supernatant fraction enriched in κ-casein is similarly easily precipitated at acid pH when the calcium chloride concentration is low, and is easily separated by filtration or low speed centrifugation. Both fractions can then be further purified, if necessary on an industrial scale.

For example, additional processing may be carried out to separate caseinomacropeptide (CMP) from the κ-casein fraction. CMP has a molecular size of around 6,500 daltons in the monomeric form, however CMP only adopts a monomeric form at acidic pH values, e.g., at pH4. At a neutral pH, CMP takes a tetromeric form and has an effective molecular size of 25,000 doltons. Therefore, by manipulation of the pH, CMP can be induced to behave as a molecule of comparable size to whey proteins, like β-lactoglobulin or, alternatively, to behave as a small peptide. This feature can be utilised to effectively separate CMP from mixtures with either native whey proteins (such as κ-casein preparations made from skimmed milk) or from other peptides (such as proteospeptone fraction found in skimmed milk or from peptides derived by non-specific breakdown of other casein fractions). The properties of the various fractions are shown below:

| **Component** | **Solubility at pH4.6** | **Retention by UF membrane 15kD NW cut-off** |
|---|---|---|
| αₛ, β- κ-casein | No | Yes |
| para-κ-casein | No | Not important |
| CMP monomer | Yes | No |
| CMP tetramer | Yes | Yes |
| Whey Protein | Yes | Yes |
| Chymosin | Yes | Yes |
| Proteose peptones | Yes | No |

The differences in properties described above can be exploited to manufacture CMP in a very pure state, and free from contaminants, including phenylalanine. Thus, the κ-casein fraction produced by the method described above can be processed to produce very pure CMP. Typically the pH of κ-casein fraction will be increased to neutral pH in order to convert the CMP to its tetrameric form. Thereafter, treatment by ultra-filtration using a membrane with the appropriate pore size - typically less than 25kD, but greater than 12kD will separate all the low molecular weight material from the caseins, whey proteins and CMP.

Acidification to pH4.6 results in conversion of the CMP back to the monomeric form and in precipitation of the other casein fractions. In this case, treatment by ultra-filtration using a membrane with the appropriate pore size - typically more than 7kD, but less than 20kD results in separate of the whey protein from CMP. CMP may be conveniently concentrated and dried, typically by freeze or spray drying. It will be appreciate that the process described above is only one of many suitable combinations - some of which may reverse the order of pH adjustment - and is given by way of example only.

The application of the principle for purification of CMP is not restricted to material derived from skimmed milk, for example the process could equally be applied to separation and purification of CMP from cheese or rennet whey. In such cases, a preliminary treatment by centrifugal separation and/or by micro-filtration (typically using a membrane with a cut-off at 1.8µ) is appropriate to remove contaminates such as curd particles, residual fat and bacteria. After this pre-treatment, the above described principles can be employed to make CMP preparations free from pHA and suitable for the nutrition of patients suffering from PKU.

At low temperatures, β-casein remains soluble at its isoelectric point, and can therefore be separated from the accompanying αₛ-caseins by their precipitation at 2 °C and pH 4.6 (JOURNAL OF AMERICAN CHEMICAL SOCIETY vol. 67, 1944, pages 1725-1731. Warner, R.C. "Separation of α- and β-casein"). β-Casein is then recovered by precipitation from the supernatant at 35 °C. This procedure is repeated until all of the β-casein is extracted from the αₛ-caseins, yielding pure fractions of αₛ- and β-caseins (Figure 3).

The procedure for selectively precipitating purified casein fractions from a caseinate solution follows essentially the same principles to those described for skim-milk, except that on adding calcium chloride, the supernatant contains only κ-, β- and αₛ-caseins (Figure 5). As mentioned previously, the caseins can be partially purified on the basis of their solubilities in ethanol (BIOCHEMISTRY vol. 9, 1970, pages 2807-2813. Talbot, B. and Waugh, D.F. "Micelle-forming characteristics of monomeric and covalent polymeric κ-caseins"). This procedure can be used to purify the κ-casein fraction. The initial κ-casein fraction is re-precipitated to remove residual calcium and selectively precipitated from ethanol solution, αₛ₁- and β-caseins remaining in the supernatant. The precipitate forms readily and can be removed by low speed centrifugation, giving a fraction enriched in κ-casein (Figure 6).

As found for skim-milk, fractionation of sodium caseinate at high pH has the advantage over previous methods involving calcium precipitation at neutral pH, or separation in the presence of chaotropic agents, that it yields all three caseinate fractions free of toxic materials, and therefore suitable for use as food ingredients. The lower calcium concentrations required at alkaline pH facilitate the subsequent recovery of the κ-casein enriched fraction without the necessity for the addition of a toxic chelating agent, or dialysis which is not easily carried out on a large scale. Similarly, the improved separation at alkaline pH of the αₛ- and β-casein precipitate from the κ-casein supernatant without the requirement for high-speed centrifugation facilitates scaling up the method for producing food-grade materials.

Prolonged treatment at alkaline pH, especially at high temperature, can lead to dephosphorylation of casein (JOURNAL OF DAIRY RESEARCH, vol. 39, 1972, pages 189-194. Manson, W. and Carolan, T. "The alkali-induced elimination of phosphate from β-casein"). The dehydroalanine formed in the caseins as a result of the dephosphorylation, and possibly also due to removal of -SH groups from cysteine, reacts with s-amino groups on lysine residues to form lysinoalanyl residues. This reaction leads to a reduction in the amount of lysine available for nutrition and to increased cross-linking and reduction in the digestibility of the caseins. Similar reactions occur when milk is heated, and there is some evidence from animal studies that lysinolanine may be harmful if fed in large quantities (JOURNAL OF NUTRITION, vol. 98, 1969, pages 45-56. De Groot, A.P. and Slump, P. "Effects of severe alkali treatment of proteins on amino acid composition and nutritive value"). It is believed that the risk to the health of humans of eating heat or alkali-treated milk proteins is small (JOURNAL OF DAIRY RESEARCH, vol.49, pages 725-736. de Koning, P.J. and van Rooijen, P.J. "Aspects of the formation of lysinoalanine in milk and milk products").

In the present work, however, we carried out a careful study of the effects of the alkaline treatment on the milk proteins, applying techniques such as anion- and cation-exchange fast protein liquid chromatography and capillary electrophoresis. These techniques have previously been successfully used to detect the analogous changes in the proteins induced by heating (MILCHWISSENSCHAFT, vol. 49, 1994, pages 125-129. Law, A.J.R., Horne, D.S., Banks, J.M. and Leaver, J. "Heat-induced changes in the whey proteins and caseins"; JOURNAL OF CHROMATOGRAPHY vol. 652, 1993, pages 207-213. de Jong, N., Visser, S. and Olieman, C. "Determination of milk proteins by capillary electrophoresis"). The changes that occur on severe alkaline treatment, such as dephosphorylation, removal of -SH and carbohydrate groups (sialic acid), all lead to a reduction in negative charge of the proteins, and these changes, which also occur on heating, can be detected by anion-exchange fast protein liquid chromatography (FPLC). Similarly, the deamination of ε-amino Lys and Arg residues that occurs on prolonged treatment at alkaline pH leads to loss of positive charge in the proteins, and these changes can be determined by cation-exchange FPLC and capillary electrophoresis at acid pH.

These techniques were used to examine caseins that had been subjected to the alkaline conditions required for selective precipitation of the casein fractions from skim-milk or sodium caseinate. Results from anion-exchange FPLC (Figure 7) show that, within the short times required to accomplish dissociation of the casein micelles in milk (5 min), there were no measurable changes in the profiles or in the relative peak areas of the individual caseins. As the separation is based on net negative charge, it appears that there were no appreciable changes in the charges on the individual caseins and that, in particular, dephosphorylation was not taking place. Results from capillary electrophoresis (Figure 8) and cation-exchange FPLC (Figure 9) similarly show that, following alkaline treatment for 5 min, there were no measurable changes in the profiles or in the relative peak areas of the caseins. Both techniques were carried out at acid pH and, as separations are based on net positive charge, show that short alkaline treatment had no effect on positively charged groups such as Lys and Arg on the caseins. The collective results show that the short treatment under alkaline conditions required for the selective precipitation of the caseins from skim-milk or sodium caseinate did not cause any chemical damage to the proteins.

During the alkaline treatment of skim-milk, β-lactoglobulin undergoes a conformational change, and subsequently becomes insoluble at its isoelectric point (JOURNAL OF THE AMERICAN CHEMICAL SOCIETY vol. 81, 1959, pages 4032-4036. Tanford, C., Bunville, L.G. and Nozaki, Y. "The reversible trans-formation of β-lactoglobulin at pH 7.5"). Under the alkaline conditions required for selective calcium precipitation of the caseins, some of the β-lactoglobulin becomes denatured but the other whey proteins, as shown by gel permeation FPLC (Figure 4), were not denatured and retained their solubilities at their isoelectric points. Previous results from reverse phase HPLC, capillary electrophoresis and tryptic digests of the proteins from skim-milk held briefly at alkaline pH showed that there were no significant changes in the chemical properties of the proteins (PATENT APPLICATION, 2001, Leaver, J. and Law, A.J.R. "Milk and cheese extraction process, including methods of extracting β-lactoglobulin and caseins from milk and milk products, and novel products thereby produced").

### Example 1

Skim-milk was warmed to 30 °C and the pH rapidly adjusted to 11.0 by the addition, with stirring, of 5M sodium hydroxide. While continuing to stir, a 0.5M solution of calcium chloride was added, the total volume of added calcium chloride being 133 ml per litre of skim-milk solution and the final concentration being 0.059M. Hydrochloric acid (5M) was then added, with stirring in order to reduce the pH of the skim-milk to 7.0. A precipitate (A) was formed which was collected either by centrifugation or allowed to settle and removed by filtration. This precipitate was rich in αₛ-and β-caseins and contained little κ-casein. The pH of the supernatant was reduced to 3.8 by the addition, with stirring of 1M HCl. A second precipitate (B) formed which was collected in the same way as precipitate A. Precipitate B contained κ-casein (60%) and β-lactoglobulin (20%) and small amounts of αₛ- and β-caseins (Figure 3). The supernatant of acid whey was partially depleted in β-lactoglobulin (Figure 4).

Precipitate A was re-suspended in water at approximately the same concentration as in the initial caseinate by vigorous stirring at 20 °C with the pH being continuously adjusted to 7.0 by the addition of 1M HCl. After complete dissolution, the solution was cooled to 2 °C and the pH was adjusted to 4.5 by the addition, with stirring, of 1M HCl. A precipitate (C) formed and, after stirring for 15 minutes at 2 °C, this was harvested from the supernatant (D) either by filtration or centrifugation at 2 °C. This precipitate was highly enriched in αₛ-casein but contained some β-casein. The amount of β-casein in this fraction was reduced by re-dissolving the precipitate in water and repeating the precipitation at 2 °C twice more. Analysis showed that more than 85% of the protein was αₛ-casein (Figure 3).

The supernatant D, was warmed to 35 °C and the pH was adjusted to 4.6 by the addition, with stirring of small volumes of 1M HCl. A precipitate (E) formed which was harvested by centrifugation or filtration in the same way as precipitate A. Analysis showed that more than 95% of the protein was β-casein (Figure 3) .

### Example 2

Sodium caseinate was dissolved in water at a concentration of 27 g/L by stirring at approximately 37 °C for 10 minutes (Figure 4). The solution was cooled to 30 °C and the pH rapidly adjusted to 11.0 by the addition, with stirring, of 5M sodium hydroxide. While continuing to stir, a 0.5M solution of calcium chloride was added, the total volume of added calcium chloride being 166 ml per litre of caseinate solution and the final concentration being 0.071M. Hydrochloric acid (5M) was then added, with stirring in order to reduce the pH of the caseinate solution to 7.0. A precipitate (A) was formed which was collected either by centrifugation or allowed to settle and removed by filtration. This precipitate was rich in αₛ- and β-caseins and contained little or no κ-casein. The pH of the supernatant was reduced to 3.8 by the addition, with stirring of 1M HCl. A second precipitate (B) formed which was collected in the same way as precipitate A. Precipitate B was highly enriched in κ-casein and contained approximately equal amounts of αₛ-, β- and κ-casein (Figure 4). Precipitate B could be further purified by dissolving in water at pH 7.0, and re-precipitating at pH 4.6 to reduce the content of calcium salt. The precipitate was re-dissolved at pH 7.0, and κ-casein selectively precipitated from 50% ethanol solution by the addition of a small amount of 2M sodium chloride in 50% ethanol, most of the αₛ₁- and β-caseins remaining in the supernatant. The precipitate formed readily and could be separated by low-speed centrifugation or filtration. The fraction was about 65% κ-casein (Figure 6) and, as toxic materials were not involved, was suitable as a food ingredient.

Precipitate A was re-suspended in water at approximately the same concentration as in the initial caseinate by vigorous stirring at 20 °C with the pH being continuously adjusted to 7.0 by the addition of 1M HCl. After complete dissolution, the solution was cooled to 2 °C and the pH was adjusted to 4.5 by the addition, with stirring, of 1M HCl. A precipitate (C) formed and, after stirring for 15 minutes at 2 °C, this was harvested from the supernatant (D) either by filtration or centrifugation at 2 °C. This precipitate was highly enriched in αₛ-casein but contained some β-casein. The amount of β-casein in this fraction was reduced by re-dissolving the precipitate in water and repeating the precipitation at 2 °C twice more. Analysis showed that more than 85% of the protein was αs-casein (Figure 5).

The supernatant D, was warmed to 35 °C and the pH was adjusted to 4.6 by the addition, with stirring of small volumes of 1M HCl. A precipitate (E) formed which was harvested by centrifugation or filtration in the same way as precipitate A. Analysis showed that more than 95% of the protein was β-casein (Figure 5).

### Applications for the purified casein fractions

The purified casein fractions can be prepared on a large scale, free of toxic materials, and can be added to foods or used for preparing new food products. The individual caseins have different structures and properties and can, therefore, be selected to give optimal functional properties for a particular purpose. β-Casein, for example, is more hydrophobic and more soluble in ethanol than the other caseins, whereas κ-casein is hydrophilic and easily precipitated from ethanol solutions. Similarly, the αₛ-caseins, which are rich in phosphate groups, readily bind divalent ions and are much more sensitive to calcium concentrations than κ-casein, which has only a single phosphate group. κ-Casein also has special properties due to its occurrence as a series of disulphide-linked polymers, the presence of hydrophilic carbohydrate side-chains, and its ability to stabilise the other caseins in aqueous solutions. The casein fractions also show different susceptibilities to different enzymes, and are suitable starting materials for preparing a wide range of peptides as described below.

During cheese ripening, the caseins undergo proteolysis and the resulting peptides and amino acids make an important contribution to the flavour of matured cheeses. In the early stages of ripening, chymosin and plasmin cause proteolysis of large and intermediate-sized peptides, whereas at later stages other proteolytic enzymes produce short peptides and ultimately amino acids. However, maturation is a slow process, and there is considerable potential for developing cheese flavours by rapid proteolysis of isolated caseins. During this process, hydrophobic peptides can be produced, especially from β-casein, that have a bitter flavour. The purified casein fractions described here can be used as starting materials permitting more control over the type of peptides formed and the flavours developed.

Controlled hydrolysis of the caseins by acidic or alkaline conditions or by limited enzymatic proteolysis yields peptides that have enhanced foaming ability, the small molecules being able to diffuse faster than the parent caseins to the interfaces. Peptides from the caseins have the advantages of being heat stable, have low allergenicity and facilitate calcium binding. Specific hydrolysates are also used to optimise the fermentation rate and increase the cell-count of certain bioactive strains of bacteria in yoghurt processing. The availability of purified fractions obtained by the present procedure facilitates the production of a wide variety of peptides having the required properties.

Although most replacement infant feeds are based on cows' milk, the concentration of total protein in human milk is lower, and the relative proportions of the individual proteins differ considerably from those in cows' milk. In particular, human milk does not contain β-lactoglobulin, and the relative proportion of αₛ-caseins is very low. In contrast, the relative proportions of α-lactalbumin, lactoferrin and of κ- and β-caseins are high in human milk. Also, β-casein which accounts for about 60 % of the total casein in human milk, occurs as a series of differently phosphorylated polypeptides, the overall degree of phosphorylation being lower than in cows' milk. It is believed that differences in composition may account for some of the problems associated with feeding cows' milk to infants. For example, the allergenicity of cows' milk has been linked in part to the presence of β-lactoglobulin. Similarly, difficulties in digestion may be due to the harder texture of the curd formed in the stomach at acid pH, when milk has less of the lower phosphorylated β-casein and more of the highly phosphorylated αₛ-caseins. In recent years, there has been a gradual change in the manufacture of infant formula towards obtaining a product that more closely resembles human milk. The large-scale preparation of individual casein fractions and whey that is partially depleted in β-lactoglobulin, as described here, could help to achieve this. Purified β-casein can be partially dephosphorylated by the action of phosphatase, and recombined with κ-casein and the whey to give milk more closely resembling human milk in protein composition.

In common with many long-chain natural or synthetic molecules, the caseins can be cross-linked to form polymers, usually by the application of heat and high pressure, followed by treatment with formalin (In CASEIN AND ITS INDUSTRIAL APPLICATIONS, 1939, pages 181-232, Reinhold Publishing Corporation, New York, Brother, G.H. "Casein Plastics"). The process was once commonly used to produce a wide range of plastics having satisfactory texture and colour but, because of the cost of raw materials and hazards of formalin, it has been replaced by methods using synthetic polymers. There is, however, a market for edible food coatings and biodegradable plastics and, by using milder cross-linking techniques involving addition of calcium at alkaline pH, it is possible to use the individual casein fractions described here to produce a range of soft plastics having suitable properties.

In recent years it has been established that the proteolytic breakdown of caseins in milk, or during digestion, gives rise to bioactive peptides that have special physiological functions. Bioactive peptides include phosphopeptides, and peptides with antimicrobial, antihypertensive, immunomodulatory, antithrombotic and opioid functions (JOURNAL OF DAIRY SCIENCE, Vol. 83, 2000, pages 1187-1195. Clare, D.A. and Swaisgood, H.E. "Bioactive milk peptides: A prospectus").

Casein phosphopeptides have been identified after trypsin release from the individual caseins and, because of the characteristic grouping of phosphoserine residues and high negative charge of the phosphate centres, are believed to function as carriers for different minerals, especially calcium (PROCEEDINGS OF 25^{th} INTERNATIONAL DAIRY CONGRESS, 1998, pages 200-208. Holt, C. "Casein structure and casein-calcium phosphate interactions". The casein phosphopeptides are mostly resistant to enzymatic proteolysis in the gut and are usually found complexed with calcium phosphate. This complex formation results in increased solubility and enhanced absorption of calcium required for bone calcification. Casein phosphopeptides also inhibit caries lesions by helping to recalcify dental enamel, and their application in the treatment of dental disease is being promoted. The individual caseins give rise to different phosphopeptides, some of which have already found applications as dietary supplements and medicines because of their ability to bind Fe, Zn and Cu, and the availability of purified casein fractions described here facilitates their preparation.

Several casein peptides with antimicrobial properties in milk have been identified, including casosidin I from αₛ₂-casein and isracidin from αₛ₁-casein B. Isracidin effectively prevents mastistis in sheep and goats, and there is considerable potential for further development of specific antibiotics of this type.

Various peptides from the caseins inhibit angiotensin converting enzyme and, therefore, act to reduce blood pressure. Of these bioactive peptides, one is a fragment of αₛ₁-casein (αₛ₁-casokinin-5) and two are obtained from β-casein (β-casokinin-7 and antihypertensive peptide).

It has also been established that casein fragments affect the immune system and the related cell proliferation responses. Of these bioactive peptides, a tripeptide of κ-casein and a peptide of β-casein have been shown to increase proliferation of peripheral blood lymphocytes.

Several peptides from the caseins have antithrombotic properties, and act by blocking the specific receptors of platelets during blood coagulation. These peptides include the glycomacropeptide, and two smaller fragments of κ-casein giving casoplatelin and thrombin inhibitory peptide, respectively.

Various studies have shown that some fragments of the caseins, the opioid peptides, have morphine-like properties. The major opioid peptides are fragments of β-casein, but they can also be obtained by pepsin hydrolysis of αₛ₁-casein. The β-casomorphins are resistant to digestive enzymes and in adults their physiological influences are limited to the gastrointestinal tract, with important effects on the intestinal transit time, amino acid uptake and water balance. In neonates, however, β-casomorphins can enter the bloodstream promoting calmness and sleep in infants. Some peptides, opioid antagonists, exert the opposite effects of the β-casomorphins, and act by suppressing the activity of enkephalin. Three of these, the casoxins, are peptides of κ-casein. The caseinomacropeptide of κ-casein also has a direct effect on the gastrointestinal tract, and inhibits gastric acid secretions. The role of bioactive peptides from caseins is an expanding area of research, especially now that there is a better understanding of the biological processes at the molecular level. The preparation of purified caseins by the present method facilitates the production of the various peptides in pure form.

The novel method of the present invention allows casein to be fractionated to yield material of hitherto unattainable purity at a markedly reduced cost and free from potential harmful reagents. In particular, the present invention permits, for the first time, preparation of material highly enriched in κ-casein.

## Claims

1. A method of obtaining purified protein fractions from milk, including the steps of:
i) increasing the pH of the milk to about pH 11;
ii) adding Calcium Chloride to selectively precipitate the fractions;
iii) decreasing the pH; and
iv) separating the fractions.

2. A method as claimed in Claim 1, wherein the fractions are αₛ-, β- and κ- casein.

3. A method as claimed in Claims 1 to 2, wherein the fractions are separated by centrifugation.

4. A method as claimed in Claims 1 to 2, wherein the fractions are separated by filtration.

5. A method as claimed in any one of the preceding Claims wherein the milk is skim milk.

6. A method as claimed in Claim 3, wherein centrifugation is carried out at low speed.

7. A method as claimed in any one of the preceding Claims, further comprising the step of carrying out precipitation at acidic pH of the supernatant obtained from centrifugation or filtration, to obtain a fraction highly enriched in κ-casein.

8. A method as claimed in Claim 7, wherein the pH is 3.8.

9. A method as claimed in any one of the preceding Claims, also comprising the steps of re-dissolving the αₛ- and β-casein components in water, and carrying out repeated selective isolelectric precipitation at acidic pH and low temperature, to obtain a fraction containing mainly αₛ-casein.

10. A method as claimed in Claim 9, wherein the water is pH 7.0 and 20°C.

11. A method as claimed in Claims 9 to 10, wherein the solution of αₛ- and β-caseins is cooled after being re-dissolved in water, and adjusted to acidic pH.

12. A method as claimed in Claim 11, wherein the solution of αₛ- and β-caseins is cooled to 2°C, and adjusted to pH4.5.

13. A method as claimed in Claims 9 to 12, wherein the procedure of re-dissolving and re-precipitation is repeated, yielding a fraction containing mainly αₛ-casein and a fraction containing mainly β-casein.

14. A method as claimed in Claims 13, wherein the supernatant obtained from the selective isoelectric precipitation is warmed, and is treated by acid precipitation to recover the fraction containing mainly β-casein.

15. A method as claimed in Claim 14, wherein the supernatant is warmed to 35ºC.

16. A method as claimed in any one of the preceding Claims, wherein the pH of the milk is increased by the addition of an alkaline solution, and decreased by the addition of an acidic solution.

17. A method as claimed in any one of the preceding Claims, wherein the pH of the milk is increased by adding sodium hydroxide.

18. A method as claimed in any one of the preceding Claims, wherein the temperature of the milk on addition of the alkaline solution is 30°C.

19. A method as claimed in any one of the preceding Claims, wherein after the step of increasing the pH of the milk, the solution is allowed to stand for a period of up to 5 minutes.

20. A method as claimed in any one of the preceding Claims, wherein the final concentration of calcium chloride in the solution is 0.059 M.

21. A method as claimed in any one of the preceding Claims, wherein the pH is decreased by addition of one or more mineral acids.

22. A method as claimed in any one of the preceding Claims, also comprising the step of increasing the pH of the fraction highly enriched in κ-casein, and carrying out ultra-filtration to separate the low molecular weight material from the caseins, whey proteins and caseinomacropeptide in the milk.

23. A method as claimed in Claim 22, wherein the pH is raised to a neutral value.

24. A method as claimed in Claims 22 to 23, wherein ultra-filtration is carried out using a membrane with a pore size less than 25kD, but greater than 12kD.

25. A method as claimed in any one of the preceding Claims, also comprising the step of lowering the pH of the fraction highly enriched in κ-casein and carrying out ultra-filtration to separate the whey protein from the caseinomacropeptide.

26. A method as claimed in Claim 25, wherein the pH is lowered to between pH4 and pH5.

27. A method as claimed in Claims 25 to 26, wherein ultra-filtration is carried out using a membrane with a pore size more than 7kD, but less than 20kD.

28. A method of obtaining purified protein fractions from a solution of sodium caseinate including the steps of:
i) increasing the pH of the sodium caseinate solution to about pH 11;
ii) adding calcium chloride to selectively precipitate the fractions;
iii) decreasing the pH; and
iv) separating the fractions.

29. A method as claimed in Claim 28, wherein the fractions are αs-, β- and κ- caseins.

30. A method as claimed in Claims 28 to 29, wherein the fractions are separated by centrifugation.

31. A method as claimed Claims 28 to 30, wherein the fractions are separated by filtration.

32. A method as claimed in Claim 31, wherein centrifugation is carried out at low speed.

33. A method as claimed in Claims 28 to 32, also comprising the step of carrying out precipitation at acidic pH of the supernatant obtained from the centrifugation or filtration, to obtain a fraction highly enriched in κ-casein.

34. A method as claimed in Claim 33, wherein the pH is 3.8.

35. A method as claimed in Claims 28 to 34, wherein the fraction enriched in κ-casein is selectively precipitated from ethanol to further enrich it in κ-casein.

36. A method as claimed in Claim 35, wherein the fraction highly enriched in κ-casein is re-dissolved in water at pH7, re-precipitated at pH4.6, and then selectively precipitated from 50% ethanol at pH7 and at 25ºC with the addition of a small amount 2NaCl in 50% ethanol.

37. A method as claimed in Claims 28 to 36, further comprising the steps of re-dissolving the αₛ- and β-casein components in water, and carrying out repeated selective isolelectric precipitation at acidic pH and low temperature to obtain a fraction containing mainly αₛ-casein.

38. A method as claimed in Claim 37, wherein the water is pH 7.0 and 20°C.

39. A method as claimed in Claims 37 to 38, wherein the solution of αₛ- and β-casein is cooled to 2ºC, and adjusted to acidic pH.

40. A method as claimed in Claim 39, wherein the solution of αₛ- and β-casein is cooled to 2ºC, and adjusted to pH4.5.

41. A method as claimed in Claims 37 to 39, wherein the supernatant obtained from the selective isolelectric precipitation is warmed, and treated by acid precipitation to recover the fraction containing mainly β casein.

42. A method as claimed in Claims 37 to 41, wherein the process of re-dissolving and re-precipitation is repeated, yielding a fraction containing mainly αₛ-casein, and a fraction containing mainly β-casein.

43. A method as claimed in any one of Claims 28 to 42, wherein the pH of the sodium caseinate solution is increased by the addition of an alkaline solution, and decreased by the addition of an acid solution.

44. A method as claimed in any one of Claims 28 to 43, wherein the pH of the sodium caseinate solution is increased to pH11 by the addition of sodium hydroxide.

45. A method as claimed in any one of Claims 28 to 44, wherein the temperature of the sodium caseinate solution on addition of the alkaline solution is 30ºC.

46. A method as described in any one of Claims 28 to 45, wherein after the step of increasing the pH of the sodium caseinate solution, the solution is allowed to stand for a period of up to 5 minutes.

47. A method as claimed in any one of Claims 28 to 46, wherein the final concentration of calcium chloride in the alkaline sodium caseinate solution will be 0.071 M.

48. A method as claimed in any one of Claims 28 to 47, wherein the pH is decreased to pH 7.0 by addition of one or more mineral acids.

49. A method as claimed in any one of Claims 28 to 48, also comprising the step of increasing the pH of the fraction highly enriched in κ-casein, and carrying out ultra-filtration to separate the low molecular weight material from the caseins, whey proteins and caseinomacropeptide in the sodium caseinate solution.

50. A method as claimed in Claim 49, wherein the pH is raised to a neutral value.

51. A method as claimed in Claims 49 to 50, wherein ultra-filtration is carried out using a membrane with a pore size less than 25kD, but greater than 12kD.

52. A method as claimed in any one of the Claims 28 to 51, also comprising the step of lowering the pH of the fraction highly enriched in κ-casein, and carrying out ultra-filtration to separate the whey protein from the caseinomacropeptide.

53. A method as claimed in Claim 52, wherein the pH is lowered to between pH 4 and pH 5.

54. A method as claimed in Claims 52 to 53, wherein ultra-filtration is carried out using a membrane with a pore size more than 7kD, but less than 20kD.

## Patentansprüche

1. Methode zum Erhalten gereinigter Proteinfraktionen aus Milch, umfassend die Schritte des:
i) Erhöhens des pH-Werts der Milch auf einen pH-Wert von etwa 11;
ii) Zusetzens von Calciumchlorid zum selektiven Ausfällen der Fraktionen;
iii) Reduzierens des pH-Werts; und
iv) Trennen der Fraktionen.

2. Methode nach Anspruch 1, wobei die Fraktionen αₛ-, β- und κ-Casein sind.

3. Methode nach Anspruch 1 bis 2, wobei die Fraktionen durch Zentrifugieren getrennt werden.

4. Methode nach Anspruch 1 bis 2, wobei die Fraktionen durch Filtrieren getrennt werden.

5. Methode nach einem der vorhergehenden Ansprüche, wobei die Milch Magermilch ist.

6. Methode nach Anspruch 3, wobei das Zentrifugieren bei geringer Geschwindigkeit durchgeführt wird.

7. Methode nach einem der vorhergehenden Ansprüche, des Weiteren umfassend den Schritt des Durchführens der Ausfällung bei saurem pH-Wert der überstehenden durch Zentrifugieren oder Filtrieren erhaltenen Flüssigkeit, um eine stark an κ-Casein angereicherte Fraktion zu erhalten.

8. Methode nach Anspruch 7, wobei der pH-Wert 3,8 beträgt.

9. Methode nach einem der vorhergehenden Ansprüche, außerdem umfassend die Schritte des erneuten Lösens der αₛ- und β-Caseinkomponenten in Wasser und Durchführens der wiederholten selektiven isoelektrischen Ausfällung bei einem sauren pH-Wert und niedriger Temperatur, um eine Fraktion zu erhalten, die hauptsächlich αₛ-Casein enthält.

10. Methode nach Anspruch 9, wobei das Wasser einen pH-Wert von 7,0 und 20 °C aufweist.

11. Methode nach Anspruch 9 und 10, wobei die Lösung der αₛ- und β-Caseine nach dem erneuten Lösen in Wasser gekühlt und auf einen sauren pH-Wert eingestellt wird.

12. Methode nach Anspruch 11, wobei die Lösung der αₛ und β-Caseine auf 2 °C gekühlt und auf einen pH-Wert von 4,5 eingestellt wird.

13. Methode nach den Ansprüchen 9 bis 12, wobei das Verfahren des erneuten Lösens und erneuten Ausfällens unter Erzeugung einer Fraktion, die hauptsächlich αₛ-Casein enthält und einer Fraktion, die hauptsächlich β-Casein enthält, wiederholt wird.

14. Methode nach Anspruch 13, wobei die überstehende Flüssigkeit, die durch die selektive isoelektrische Ausfällung erhalten wird, erwärmt und durch saure Ausfällung behandelt wird, um die Fraktion zu gewinnen, die hauptsächlich β-Casein enthält.

15. Methode nach Anspruch 14, wobei die überstehende Flüssigkeit auf 35 °C erwärmt wird.

16. Methode nach einem der vorhergehenden Ansprüche, wobei der pH-Wert der Milch durch Zusetzen einer alkalischen Lösung erhöht und durch Zusetzen einer sauren Lösung reduziert wird.

17. Methode nach einem der vorhergehenden Ansprüche, wobei der pH-Wert der Milch durch Zusetzen von Natriumhydroxid erhöht wird.

18. Methode nach einem der vorhergehenden Ansprüche, wobei die Temperatur der Milch auf das Zusetzen der alkalischen Lösung 30 °C beträgt.

19. Methode nach einem der vorhergehenden Ansprüche, wobei nach dem Schritt des Erhöhens des pH-Werts der Milch man die Lösung für eine Zeitspanne von bis zu 5 Minuten stehen lässt.

20. Methode nach einem der vorhergehenden Ansprüche, wobei die Endkonzentration des Calciumchlorids in der Lösung 0,059 M beträgt.

21. Methode nach einem der vorhergehenden Ansprüche, wobei der pH-Wert durch Zusetzen einer oder mehrerer Mineralsäuren reduziert wird.

22. Methode nach einem der vorhergehenden Ansprüche, außerdem umfassend den Schritt des Erhöhens des pH-Werts der stark mit κ-Casein angereicherten Fraktion und das Durchführen der Ultrafiltrierung zum Trennen des niedermolekularen Materials von den Caseinen, Molkeproteinen und Caseinmakropeptid in der Milch.

23. Methode nach Anspruch 22, wobei der pH-Wert auf einen neutralen Wert angehoben wird.

24. Methode nach Anspruch 22 bis 23, wobei das Ultrafiltrieren unter Anwendung einer Membran mit einer Porengröße von weniger als 25 kD aber mehr als 12 kD durchgeführt wird.

25. Methode nach einem der vorhergehenden Ansprüche, außerdem umfassend den Schritt des Reduzierens des pH-Werts der stark mit κ-Casein angereicherten Fraktion und das Durchführen der Ultrafiltrierung zum Trennen des Molkeproteins von dem Caseinmakropeptid.

26. Methode nach Anspruch 25, wobei der pH-Wert auf einen pH-Wert zwischen 4 und 5 reduziert wird.

27. Methode nach den Ansprüchen 25 bis 26, wobei die Ultrafiltrierung unter Anwendung einer Membran mit einer Porengröße von mehr als 7 kD jedoch weniger als 20 kD durchgeführt wird.

28. Methode zum Erhalten gereinigter Proteinfraktionen aus einer Lösung von Natriumcaseinat umfassend die Schritte des:
i) Erhöhens des pH-Werts der Natriumcaseinatlösung auf einen pH-Wert von etwa 11;
ii) Zusetzens von Calciumchlorid zum selektiven Ausfällen der Fraktionen;
iii) Reduzierens des pH-Werts; und
iv) Trennen der Fraktionen.

29. Methode nach Anspruch 28, wobei die Fraktionen αₛ-, β- und κ-Caseine sind.

30. Methode nach den Ansprüchen 28 bis 29, wobei die Fraktionen durch Zentrifugieren getrennt werden.

31. Methode nach den Ansprüchen 28 bis 30, wobei die Fraktionen durch Filtrieren getrennt werden.

32. Methode nach Anspruch 31, wobei das Zentrifugieren bei geringer Geschwindigkeit durchführt wird.

33. Methode nach den Ansprüchen 28 bis 32, außerdem umfassend den Schritt des Durchführens der Ausfällung bei saurem pH-Wert der überstehenden durch Zentrifugieren oder Filtrieren erhaltenen Flüssigkeit, um eine stark an κ-Casein angereicherte Fraktion zu erhalten.

34. Methode nach Anspruch 33, wobei der pH-Wert 3,8 beträgt.

35. Methode nach den Ansprüchen 28 bis 34, wobei die mit κ-Casein angereicherte Fraktion selektiv aus Ethanol ausgefällt wird, um sie noch weiter an κ-Casein anzureichern.

36. Methode nach Anspruch 35, wobei die stark mit κ-Casein angereicherte Fraktion erneut in Wasser bei einem pH-Wert von 7 gelöst, erneut bei einem pH-Wert von 4,6 ausgefällt und dann selektiv aus 50 % Ethanol bei einem pH-Wert von 7 und bei 25 °C unter Zusetzen einer kleinen Menge 2 NaCl in 50 % Ethanol ausgefällt wird.

37. Methode nach den Ansprüchen 28 bis 36, des Weiteren umfassend die Schritte des erneuten Lösens der αₛ und β-Caseinkomponenten in Wasser und Durchführens der wiederholten selektiven isoelektrischen Ausfällung bei einem sauren pH-Wert und niedriger Temperatur, um eine Fraktion zu erhalten, die hauptsächlich αₛ-Casein enthält.

38. Methode nach Anspruch 37, wobei das Wasser einen pH-Wert von 7,0 und 20 °C aufweist.

39. Methode nach den Ansprüchen 37 bis 38, wobei die Lösung von αₛ- und β-Casein auf 2 °C gekühlt und auf einen sauren pH-Wert eingestellt wird.

40. Methode nach Anspruch 39, wobei die Lösung von αₛ und β-Casein auf 2 °C gekühlt und auf einen pH-Wert von 4,5 eingestellt wird.

41. Methode nach den Ansprüchen 37 bis 39, wobei die überstehende Flüssigkeit, die durch die selektive isoelektrische Ausfällung erhalten wird, erwärmt und durch saure Ausfällung behandelt wird, um die Fraktion zu gewinnen, die hauptsächlich β-Casein enthält.

42. Methode nach den Ansprüchen 37 bis 41, wobei das Verfahren des erneuten Lösens und erneuten Ausfällens unter Erzeugung einer Fraktion, die hauptsächlich αₛ-Casein enthält und einer Fraktion, die hauptsächlich β-Casein enthält, wiederholt wird.

43. Methode nach einem der Ansprüche 28 bis 42, wobei der pH-Wert der Natriumcaseinatlösung durch Zusetzen einer alkalischen Lösung erhöht und durch Zusetzen einer saueren Lösung reduziert wird.

44. Methode nach einem der vorhergehenden Ansprüche 28 bis 43, wobei der pH-Wert der Natriumcaseinatlösung durch Zusetzen von Natriumhydroxid auf einen pH-Wert von 11 erhöht wird.

45. Methode nach einem der Ansprüche 28 bis 44, wobei die Temperatur der Natriumcaseinatlösung nach Zusetzen der alkalischen Lösung 30 °C beträgt.

46. Methode wie in einem der Ansprüche 28 bis 45 beschrieben, wobei nach dem Schritt des Erhöhens des pH-Werts der Natriumcaseinatlösung man die Lösung für eine Zeitspane von bis zu 5 Minuten stehen lässt.

47. Methode nach einem der Ansprüche 28 bis 46, wobei die Endkonzentration an Calciumchlorid in der alkalischen Natriumcaseinatlösung 0,071 M beträgt.

48. Methode nach einem der Ansprüche 28 bis 47, wobei der pH-Wert durch Zusetzen einer oder mehrerer Mineralsäuren auf einen pH-Wert von 7,0 reduziert wird.

49. Methode nach einem der Ansprüche 28 bis 48, außerdem umfassend den Schritt des Erhöhens des pH-Werts der stark mit κ-Casein angereicherten Fraktion und des Durchführens der Ultrafiltrierung zum Trennen des niedermolekularen Materials von den Caseinen, Molkeproteinen und Caseinmakropeptid in der Natriumcaseinatlösung.

50. Methode nach Anspruch 49, wobei der pH-Wert auf einen neutralen Wert angehoben wird.

51. Methode nach den Ansprüchen 49 bis 50, wobei das Ultrafiltrieren unter Anwendung einer Membran mit einer Porengröße von weniger als 25 kD aber mehr als 12 kD durchgeführt wird.

52. Methode nach einem der Ansprüche 28 bis 51, außerdem umfassend den Schritt des Reduzierens des pH-Werts der stark mit κ-Casein angereicherten Fraktion und das Durchführen der Ultrafiltrierung zum Trennen des Molkeproteins von dem Caseinmakropeptid.

53. Methode nach Anspruch 52, wobei der pH-Wert auf einen pH-Wert zwischen 4 und 5 reduziert wird.

54. Methode nach den Ansprüchen 52 bis 53, wobei das Ultrafiltrieren unter Anwendung einer Membran mit einer Porengröße von mehr als 7 kD aber weniger als 20 kD durchgeführt wird.

## Revendications

1. Procédé permettant d'obtenir des fractions de protéine purifiées à partir de lait, comprenant les étapes suivantes :
i) l'augmentation du pH du lait à environ pH 11;
ii) l'ajout de chlorure de calcium pour précipiter de manière sélective les fractions;
iii) la réduction du pH, et
iv) la séparation des fractions.

2. Procédé suivant la revendication 1, dans lequel les fractions sont des caséines αₛ, β et κ.

3. Procédé suivant les revendications 1 et 2, dans lequel les fractions sont séparées par centrifugation.

4. Procédé suivant les revendications 1 et 2, dans lequel les fractions sont séparées par filtration.

5. Procédé suivant l'une quelconque des revendications précédentes, dans lequel le lait est du lait écrémé.

6. Procédé suivant la revendication 3, dans lequel la centrifugation est exécutée à faible vitesse.

7. Procédé suivant l'une quelconque des revendications précédentes, comprenant en outre l'étape d'exécution de la précipitation à un pH acide du surnageant obtenu par centrifugation ou filtration, pour obtenir une fraction fortement enrichie en caséine κ.

8. Procédé suivant la revendication 7, dans lequel le pH est 3,8.

9. Procédé suivant l'une quelconque des revendications précédentes, comprenant également les étapes de re-dissolution des composantes caséines αₛ et β dans de l'eau, et d'exécution d'une précipitation isoélectrique sélective répétée à un pH acide et à faible température, pour obtenir une fraction contenant principalement de la caséine αₛ.

10. Procédé suivant la revendication 9, dans lequel l'eau est à pH 7 et à 20 °C.

11. Procédé suivant les revendications 9 et 10, dans lequel la solution de caséines αₛ et β est refroidie après avoir été re-dissoute dans de l'eau, et ajustée à un pH acide.

12. Procédé suivant la revendication 11, dans lequel la solution de caséines αₛ et β est refroidie à 2 °C, et ajustée à pH 4,5.

13. Procédé suivant les revendications 9 à 12, dans lequel la procédure de re-dissolution et de re-précipitation est répétée, produisant une fraction contenant principalement de la caséine αₛ et une fraction contenant principalement de la caséine β.

14. Procédé suivant la revendication 13, dans lequel le surnageant obtenu par la précipitation isoélectrique sélective est chauffé, et est traité par précipitation acide pour récupérer la fraction contenant principalement de la caséine β.

15. Procédé suivant la revendication 14, dans lequel le surnageant est chauffé à 35 °C.

16. Procédé suivant l'une quelconque des revendications précédentes, dans lequel le pH du lait est augmenté par l'ajout d'une solution alcaline, et diminué par l'ajout d'une solution acide.

17. Procédé suivant l'une quelconque des revendications précédentes, dans lequel le pH du lait est augmenté par l'addition d'hydroxyde de sodium.

18. Procédé suivant l'une quelconque des revendications précédentes, dans lequel la température du lait lors de l'ajout de la solution alcaline est 30 °C.

19. Procédé suivant l'une quelconque des revendications précédentes, dans lequel après l'étape d'augmentation du pH du lait, on laisse reposer la solution pendant un laps de temps pouvant aller jusqu'à 5 minutes.

20. Procédé suivant l'une quelconque des revendications précédentes, dans lequel la concentration finale de chlorure de calcium dans la solution est 0,059 M.

21. Procédé suivant l'une quelconque des revendications précédentes, dans lequel le pH est réduit par l'ajout d'un ou de plusieurs acides minéraux.

22. Procédé suivant l'une quelconque des revendications précédentes, comprenant également l'étape d'augmentation du pH de la fraction fortement enrichie en caséine κ, et d'exécution d'une ultrafiltration pour séparer la matière de faible poids moléculaire des caséines, les protéines lactosériques et le caséinomacropeptide dans le lait.

23. Procédé suivant la revendication 22, dans lequel le pH est élevé à une valeur neutre.

24. Procédé suivant les revendications 22 et 23, dans lequel l'ultrafiltration est exécutée en utilisant une membrane présentant une porosité inférieure à 25 kD, mais supérieure à 12 kD.

25. Procédé suivant l'une quelconque des revendications précédentes, comprenant également l'étape d'abaissement du pH de la fraction fortement enrichie en caséine κ et d'exécution d'une ultrafiltration pour séparer la protéine lactosérique du caséinomacropeptide.

26. Procédé suivant la revendication 25, dans lequel le pH est abaissé à une valeur comprise entre pH 4 et pH 5.

27. Procédé suivant les revendications 25 et 26, dans lequel l'ultrafiltration est exécutée en utilisant une membrane présentant une porosité supérieure à 7 kD, mais inférieure à 20 kD.

28. Procédé permettant d'obtenir des fractions de protéine purifiées à partir d'une solution de caséinate de sodium comprenant les étapes suivantes :
i) l'augmentation du pH de la solution de caséinate de sodium à environ pH 11 ;
ii) l'ajout de chlorure de calcium pour précipiter de manière sélective les fractions ;
iii) la réduction du pH, et
iv) la séparation des fractions.

29. Procédé suivant la revendication 28, dans lequel les fractions sont des caséines αₛ, β et κ.

30. Procédé suivant les revendications 28 et 29, dans lequel les fractions sont séparées par centrifugation.

31. Procédé suivant les revendications 28 à 30, dans lequel les fractions sont séparées par filtration.

32. Procédé suivant la revendication 31, dans lequel la centrifugation est exécutée à faible vitesse.

33. Procédé suivant les revendications 28 à 32, comprenant en outre l'étape d'exécution d'une précipitation à un pH acide du surnageant obtenu par centrifugation ou filtration, pour obtenir une fraction fortement enrichie en caséine κ.

34. Procédé suivant la revendication 33, dans lequel le pH est 3,8.

35. Procédé suivant les revendications 28 à 34, dans lequel la fraction enrichie en caséine κ est précipitée de manière sélective à partir d'éthanol pour encore l'enrichir en caséine κ.

36. Procédé suivant la revendication 35, dans lequel la fraction fortement enrichie en caséine κ est re-dissoute dans de l'eau à pH 7, re-précipitée à pH 4,6, puis précipitée de manière sélective à partir d'éthanol à 50% à pH 7 et à 25 °C avec l'ajout d'une petite quantité de 2NaCl dans de l'éthanol à 50%.

37. Procédé suivant les revendications 28 à 36, comprenant en outre les étapes de re-dissolution des composantes caséine αₛ et β dans de l'eau, et d'exécution d'une précipitation isoélectrique sélective répétée à un pH acide et à faible température pour obtenir une fraction contenant principalement de la caséine αₛ.

38. Procédé suivant la revendication 37, dans lequel l'eau est à pH 7 et à 20 °C.

39. Procédé suivant les revendications 37 et 38, dans lequel la solution de caséines αₛ et β est refroidie à 2 °C, et ajustée à un pH acide.

40. Procédé suivant la revendication 39, dans lequel la solution de caséines αₛ et β est refroidie à 2 °C, et ajustée à pH 4,5.

41. Procédé suivant les revendications 37 à 39, dans lequel le surnageant obtenu par la précipitation isoélectrique sélective est chauffé, et traité par précipitation acide pour récupérer la fraction contenant principalement de la caséine β.

42. Procédé suivant les revendications 37 à 41, dans lequel le processus de re-dissolution et de re-précipitation est répété, produisant une fraction contenant principalement de la caséine αₛ, et une fraction contenant principalement de la caséine β.

43. Procédé suivant l'une quelconque des revendications 28 à 42, dans lequel le pH de la solution de caséinate de sodium est augmenté par l'ajout d'une solution alcaline, et réduit par l'ajout d'une solution acide.

44. Procédé suivant l'une quelconque des revendications 28 à 43, dans lequel le pH de la solution de caséinate de sodium est augmenté à pH 11 par l'addition d'hydroxyde de sodium.

45. Procédé suivant l'une quelconque des revendications 28 à 44, dans lequel la température de la solution de caséinate de sodium lors de l'ajout de la solution alcaline est 30 °C.

46. Procédé suivant l'une quelconque des revendications 28 à 45, dans lequel, après l'étape d'augmentation du pH de la solution de caséinate de sodium, on laisse reposer la solution pendant un laps de temps pouvant aller jusqu'à 5 minutes.

47. Procédé suivant l'une quelconque des revendications 28 à 46, dans lequel la concentration finale de chlorure de calcium dans la solution de caséinate de sodium alcaline sera 0,071 M.

48. Procédé suivant l'une quelconque des revendications 28 à 47, dans lequel le pH est réduit à pH 7 par l'ajout d'un ou de plusieurs acides minéraux.

49. Procédé suivant l'une quelconque des revendications 28 à 48, comprenant également l'étape d'augmentation du pH de la fraction fortement enrichie en caséine κ, et d'exécution d'une ultrafiltration pour séparer la matière de faible poids moléculaire des caséines, les protéines lactosériques et le caséinomacropeptide dans la solution de caséinate de sodium.

50. Procédé suivant la revendication 49, dans lequel le pH est élevé à une valeur neutre.

51. Procédé suivant les revendications 49 et 50, dans lequel l'ultrafiltration est exécutée en utilisant une membrane présentant une porosité inférieure à 25 kD, mais supérieure à 12 kD.

52. Procédé suivant l'une quelconque des revendications 28 à 51, comprenant également l'étape d'abaissement du pH de la fraction fortement enrichie en caséine κ, et d'exécution d'une ultrafiltration pour séparer la protéine lactosérique du caséinomacropeptide.

53. Procédé suivant la revendication 52, dans lequel le pH est abaissé à une valeur comprise entre pH 4 et pH 5.

54. Procédé suivant les revendications 52 et 53, dans lequel l'ultrafiltration est exécutée en utilisant une membrane présentant une porosité supérieure à 7 kD, mais inférieure à 20 kD.
